# EUROPEAN PATENT SPECIFICATION

(11) **EP 2 903 508 B1**
(45) Date of publication and mention of the grant of the patent: **19.04.2017**
(21) Application number: 13774116.1
(22) Date of filing: 02.10.2013
(51) Int. Cl.: A61B 5/0402, A61B 5/11, A61B 5/0245, A61B 5/00

(54) **METHODS AND ARRANGEMENTS ENABLING REDUCTION OF INFLUENCE OF PHYSICAL ACTIVITY AND/OR INFLUENCE DUE TO MENTAL STRESS IN ELECTROCARDIOGRAM-BASED MEASUREMENTS**
VERFAHREN UND ANORDNUNGEN, DIE EINE REDUKTION DES EINFLUSSES PHYSISCHER AKTIVITÄT UND/ODER DES EINFLUSSES AUFGRUND GEISTIGEN STRESSES BEI ELEKTROKARDIOGRAMM-BASIERTEN MESSUNGEN ERMÖGLICHEN
PROCÉDÉS ET ASSEMBLAGES PERMETTANT LA RÉDUCTION DE L'INFLUENCE DE L'ACTIVITÉ PHYSIQUE ET/OU L'INFLUENCE DUE AU STRESS MENTAL DANS DES MESURES BASÉES SUR L'ELECTROCARDIOGRAMME

(30) Priority: 02.10.2012 US 201261708687 P
(43) Date of publication of application: 12.08.2015
(73) Proprietor: Linkura AB, 58330 Linköping (SE)
(72) Inventor: BENOSMANE, Mourad, Tlemcen, 13000 (DZ)
(74) Representative: Valea AB
(86) International application number: PCT/EP2013/070536
(87) International publication number: WO 2014/053538

(56) References cited:
- US-A1- 2006 287 608
- US-A1- 2007 032 733
- US-A1- 2011 224 503
- US-A1- 2012 123 232
- US-A1- 2012 143 031

## Description

### TECHNICAL FIELD

Embodiments herein relate to methods and devices for estimating physical activity of an individual based on electrocardiogram.

### BACKGROUND

During adolescence and adulthood a sedentary lifestyle is often adopted and is a major concern for public health. Whereas studies have shown that at the age of nine, 97% of children of a European population meet the activity recommendations, only 82% of the boys and 62% of the girls meet these recommendations at the age of 15. The consequence of low level activity is its association with several diseases, such as cardiovascular disease, diabetes mellitus type II, osteoporosis, obesity, and some cancers such as colon or breast cancer. The dramatic increase in the prevalence of overweight and obesity over the past decades is related to lower levels of physical activity. Moreover, many studies showed that individuals who are physically active enjoy better health and have a higher degree of independence than those who are sedentary. Therefore, accurate assessment of physical activity is critically important and physical activity monitors are being used in rehabilitation involving gait and locomotion, addressing obesity prevention and treatment, sleep disorders, sports, fitness and performance enhancement training, and other health outcomes.

US 2012143031 (A1) is about sensed body electrical information where electrodes of a subcutaneous monitoring system receive body electrical signals that indicate both cardiac and non-cardiac muscle activity. Exemplary systems and methods that detect non-cardiac muscle activity information in sensed body electrical waveforms provide a diagnostic tool for monitoring physical activity level over time in patients that have subcutaneous monitoring systems. In an illustrative embodiment, systems and methods for presenting patient activity information in a graphical format over intervals of time include processing ECG waveform information to identify and to accumulate non-cardiac muscular activity information during each of the intervals of time.

US 2007032733 (A1) relates to ECG-derived sleep disordered breathing monitoring, detection and classification. An apparatus is disclosed for detecting sleep disordered breathing (SDB), cardiac events and/or heart rate variability (HRV) in a subject from a physiological electrocardiogram (ECG) signal. The apparatus includes means for monitoring the ECG signal and means for extracting from the ECG signal parameters indicative of the SDB, cardiac events and/or HRV.

US 2006287608 (A1) discloses an electromyography for the detection of electromyography signals on moving subjects. An apparatus for the detection includes signal sensors and movement sensors applied to the subject. Electromyography signals are integrated with functional information concerning the patient's motor activity, so as to be able to create an exact correlation between the individual steps of each specific patient movement or action and the corresponding muscle activity detected by the sensors.

US 2012123232 (A1) is about determining heart rate variability using wavelet transformation and extract features that can accurately identify various states of the cardiovascular system. It is stated that this e.g. can be used to estimate the extent of blood volume loss, distinguish blood volume loss from physiological activities associated with exercise, and predict the presence and extent of cardiovascular disease in general.

### SUMMARY

An object of embodiments herein is to provide an improved or at least alternative way of assessing physical activity.

According to a first aspect of embodiments herein, the object is achieved by a method, performed by a device, for estimating physical activity of an individual based on electrocardiogram. The device receives an electrocardiogram signal originating from the individual. The device identifies a non-cardiac muscle signal in the received electrocardiogram signal, wherein the identifying of the non-cardiac muscle signal comprises measuring a degree of fluctuations in the electrocardiogram signal excluding fluctuations of QRS complexes. Further, the device estimates a presence or a level of physical activity of the individual, wherein the estimated presence or level of physical activity corresponds to an estimated measure of heart activity. The estimation being based on the identified non-cardiac muscle signal and on an established relationship between non-cardiac muscle signal levels and measures of physical activity, which measures comprise measures of heart activity. Moreover, the device obtains a measure of heart activity from the electrocardiogram signal and compares with the estimated measure of heart activity, thereby enabling reduction of influence of physical activity in the measure of heart activity and/or due to mental stress of the individual.

According to a second aspect of embodiments herein, the object is achieved by a method, performed by a device, for supporting estimation of physical activity of an individual based on electrocardiogram. The device receives an electrocardiogram signal originating from the individual when the individual was subjected to a predetermined physical activity. Then the device identifies a non-cardiac muscle signal in the received electrocardiogram signal, wherein the identification of the non-cardiac muscle signal comprises measuring a degree of fluctuations in the electrocardiogram signal excluding fluctuations of QRS complexes. Further, the device establishes, by using linear regression, a relationship between signal levels of the identified non-cardiac muscle signal and measures of the predetermined physical activity, which measures comprise measures of heart activity extracted from the electrocardiogram signal.

According to a third aspect of embodiments herein, the object is achieved by a computer program comprising code that when being loaded into a device performs the method according to the first or second aspect.

According to a fourth aspect of embodiments herein, the object is achieved by a data carrier comprising the computer program according to the third aspect.

According to a fifth aspect of embodiments herein, the object is achieved by a device for estimating physical activity of an individual based on electrocardiogram. The device comprises a receiving port configured to receive an electrocardiogram signal originating from the individual. The device also comprises an identifying circuitry configured to identify a non-cardiac muscle signal in the received electrocardiogram signal, and an estimating circuitry configured to estimate a presence or a level of physical activity of the individual. Said estimated presence or level of physical activity corresponds to an estimated measure of heart activity and which estimation is based on the identified non-cardiac muscle signal and on an established relationship between non-cardiac muscle signal levels and measures of physical activity, which measures comprise measures of heart activity. The identifying circuitry is further configured to measure a degree of fluctuations in the electrocardiogram signal excluding fluctuations of QRS complexes. The device additionally comprises an obtaining and comparing circuitry configured to obtain a measure of heart activity from the electrocardiogram signal and to compare the obtained measure with the estimated measure of heart activity. Thereby it is enabled reduction of influence of physical activity in the measure of heart activity and/or due to mental stress of the individual.

According to a sixth aspect of embodiments herein, the object is achieved by a device for supporting estimation of physical activity of an individual based on electrocardiogram. The device comprises a receiving port, configured to receive an electrocardiogram signal originating from the individual when the individual was subjected to a predetermined physical activity. The device also comprises an identifying circuitry configured to identify a non-cardiac muscle signal in the received electrocardiogram signal. The identifying circuitry is further configured to measure a degree of fluctuations in the electrocardiogram signal excluding fluctuations of QRS complexes. The device additionally comprises an establishing circuitry configured to use linear regression to establish a relationship between signal levels of the identified non-cardiac muscle signal and measures of the predetermined physical activity, which measures comprise measures of heart activity extracted from the electrocardiogram signal.

For example, in view of the above discussed different aspects of embodiments herein, the level of physical activity may be estimated from the established relationship and the electrocardiogram (ECG) signal, and heart rate be determined from the same electrocardiogram signal. The estimated level of physical activity may correspond to an estimated heart rate independent of mental stress. By e.g. comparing the heart rate determined from the ECG signal with the estimated heart rate, heart rate resulting from mental stress may be identified and/or determined. Hence it is e.g. enabled, based on the electrocardiogram signal, to conveniently determine and/or distinguish heart rate resulting from physical activity and/or from mental stress.

### BRIEF DESCRIPTION OF THE DRAWINGS

Examples of embodiments herein are described in more detail with reference to the appended schematic drawings, in which:
Figure 1 is a schematic diagram depicting a chest strap with electrodes for ECG signal registration in a most used position.
Figure 2 illustrates a ECG signal with non-cardiac muscle activity.
Figure 3 illustrates (a) ECG signal with a non-cardiac muscle signal segment, (b) RMS value of a detail number two (D2), (c) RMS value of a detail number one (D1).
Figure 4 illustrates (a) Subject speed vs. ECG-non-cardiac muscle signal, and (b) Subject speed vs. heart rate (Walking at 5 and 7 while running at 8 km/h)
Figure 5 illustrates an ECG Non-Cardiac signal level vs. Heart Rate (HR). The line is a least squares regression line.
Figure 6 is a flow chart illustrating a method according to embodiments herein.
Figure 7 is a schematic block diagram illustrating a device according to embodiments herein.
Figure 8 is a flow chart illustrating another method according to embodiments herein.
Figure 9 is a schematic block diagram illustrating another device according to embodiments herein.

### DESCRIPTION

Before presenting embodiments herein and as part of the development towards embodiments herein, a study underlying embodiments herein will be described.

### I. Introduction of a study

### A. Physical activity

Physical activity (PA) is conventionally defined as "any bodily movement produced by the contraction of skeletal muscle that increases energy expenditure above a basal level". Physiologically, skeletal muscular contractions can be classified according to either length changes or force levels as concentric, eccentric, or isometric (static). In many activities, all three types of muscle action may occur in the execution of a smooth and coordinated movement. PA is a complex time variant parameter that can be classified quantitatively by its frequency (number of PA events in a specific period), duration (amount of time), and intensity (physiological effort) but also qualitatively into major categories of sedentary behaviours, locomotion, work, leisure, and exercise. Finally, it can be classified contextually by dimensions of time and place, position, or posture. Several monitoring methods are available to measure physical activity. The selection of the evaluation tool depends on the PA variable of interest, study objectives, characteristics of the target population, and feasibility in terms of cost and management.

In general, most activity measurements using electronic wearable monitors are more focused on the quantification rather than on the qualification (classification) of physical activity. The measurement of contextual factors of physical activity is just beginning. The quantitative assessment of physical activity using wearable monitors is grounded in the measurement of energy expenditure (EE). Devices are calibrated and validated against EE measured by the reference methods (calorimetry or the double labeled water). One of the most used methods to estimate PA is the recording of the ECG signal using ECG-chest strap comprising electrodes. Processing the ECG signal, to record the RR intervals and to estimate the heart rate (HR) in order to measure PA since heart activity is affected by PA. As should be known to the skilled person, an RR interval refers to the time duration between successive R waves, which typically are the highest waves in an ECG signal. However, a potential shortcoming of this technique is that it is not possible to differentiate increases in the HR due to physical activity or to stress respectively, especially when the increase in HR is modest. The aim is to extend the RR interval analysis through the involvement of muscle activity recorded by the ordinary ECG electrodes, to extract more information that can be used to measure PA without being influenced by the mental activity. Ordinary electrodes in the context of ECG typically comprise electrodes for receiving bioelectric signals by contact with a body surface, typically an outer skin surface, or more particularly electrodes for recording bioelectric potentials by non-invasive contact with an outer body surface of an individual.

### B. ECG and non-cardiac muscle activity

ECG is a recording of the small (<mV) surface potentials generated during heart muscles activity. However, the surface ECG electrodes record both cardiac muscle electrical potentials as well non-cardiac muscle activity. Depending on the electrode size different muscle groups have impact on the recorded ECG signal, especially the trunk muscles. Trunk muscles have been divided into two muscle systems: the global system enabling movements and the local system ensuring stability. There are two distinct types of activation patterns: Local system muscles are permanently active at low levels, independent of movements. This low level of muscle activity appears as very low non-cardiac muscle signal amplitude present on the ECG signal during rest period. Conversely, muscles of the global system act to initiate movements leading to movement dependent phasic activation patterns. Recently, the global system was subdivided further into the global stabilizing and the global mobilizing systems respectively. Global stabilizers complement the function of the local system by controlling and limiting movements by means of eccentric activation characteristic. Global mobilizers initiate movements. The most active muscles that affect the ECG signal when electrodes are located at positions usually used for heart rate monitoring and used in the study described herein are the rectus abdominus (RA) and the oblique externus muscles (OE). RA is global mobilizer, whereas OE belongs to the global stabilizers.

In the study described herein, an extended method is proposed to measure physical activity. The extended method is based on the identification of the non-cardiac muscle activity information, generated by the trunk muscles, where the ECG surface electrodes are located, in order to generate ECG non-cardiac muscle signal level and compare it with heart rate since heart rate is an accurate method to estimate PA intensity, in particularly if its value is not influenced by mental activity.

### II. Method of the study

### A. Subject

One individual (Body mass = 86 kg, Height = 186 cm, Age = 25 yrs, and BMI= 24 kg/m²) completed the study. The subject was healthy, with no evidence of past or present disorders with no intake of drugs known to affect energy metabolism, a balanced diet, and non-smoker. The participant was encouraged to maintain their normal daily activity and food intake.

### B. Procedure

The subject performed 6 minutes each of the following eight exercises on a motorized treadmill: from slow walking (3, 4, and 5 km/h), fast walking (6,7 km/h), and jogging (8,9 and 10 km/h). Between each walking exercise, there is a 5 minutes rest period and 15 minutes between the running period.

The order of the exercises was from the lowest speed to the highest. The subject should not be under stress during the whole experience. In the analysis a 3 seconds epoch was used and both HR and non-cardiac muscle activity levels were expressed as average amplitude for the 3 last minutes of each exercise. Least square linear regression was used to establish the relationship between non-cardiac muscle signals and HR expressed as average amplitude per 3 seconds epoch.

The subject was asked to refrain from exercise and caffeine intake less than 4 h before the test. The same experimental procedure was repeated during 5 days following the same instructions. A total of 240 minutes of exercise were performed.

### C. Instrumentation

### ECG monitor

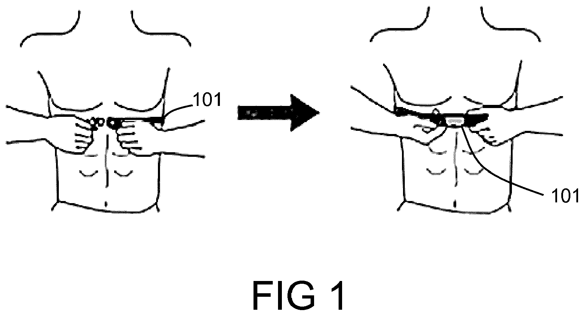

An ECG chest-strap-type of device, i.e. a device with electrodes for ECG signal registration comprised in a chest strap, similar as the chest-strap 101 illustrated in **Figure 1****,** was used to record the ECG signal. The used ECG chest-strap-type of device was a small wearable ECG device having a sampling frequency of 500 Hz and the passive first order high pass and low pass filters cut-off frequency are 0.3-240 Hz (-3db) respectively. The chest strap as such was a Polar Soft strap that was used with smart fusion of soft fabrics and adaptive electrodes to make it extremely sensitive to the skin electrical potentials.

### Electrodes positions

To find the best position of the bipolar electrodes, the ECG signal was recorded and a position of the ECG bipolar chest strap was used which gave the highest signal amplitude, found under the pectoral muscles because of its close proximity to the heart, see e.g. the location illustrated in Figure 1.

### Data processing

The ECG signal sensed by electrodes is then processed digitally in order to identify signals originating from non-cardiac muscles activity.

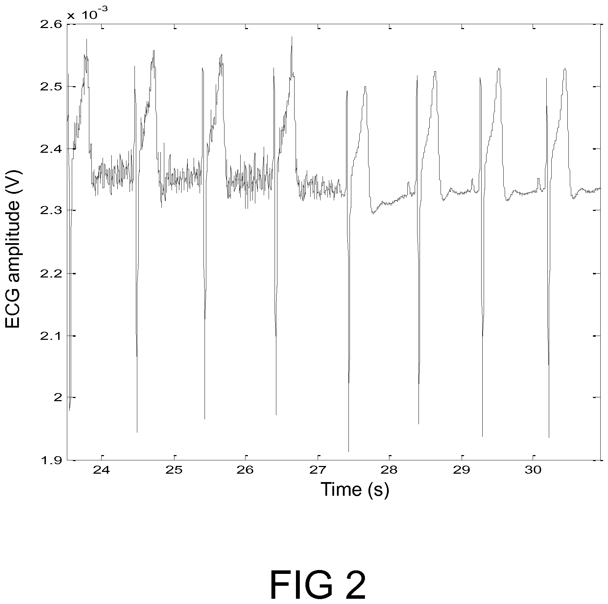

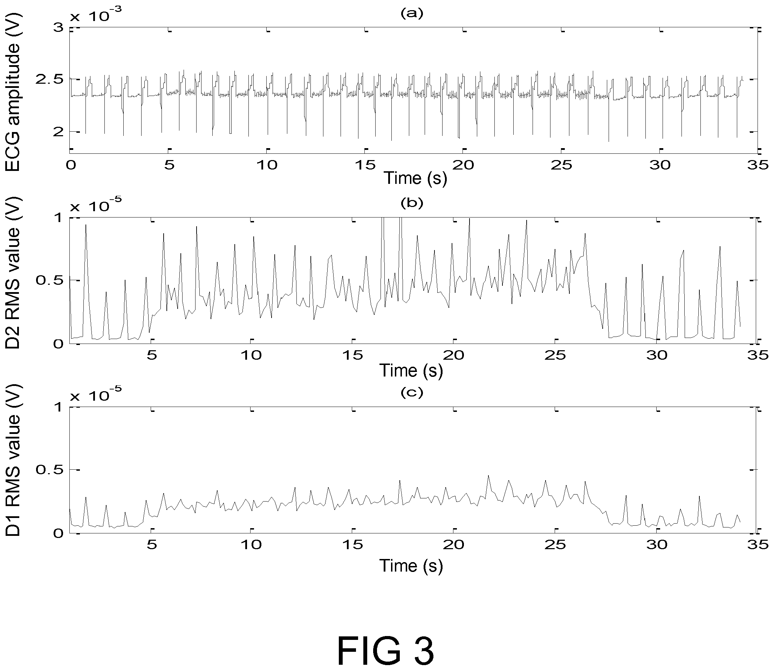

Non-cardiac muscle signals sensed by ECG electrodes located under the pectorals muscles mainly originate from muscle tissues that the electrodes are overlying. In the recorded ECG, non-cardiac-muscle signals appear as high frequency fluctuations which vary faster than ECG waves. An example of non-cardiac muscle signal in a recorded ECG is shown in **Figure 2****.** Non-cardiac muscle signals in the recorded ECG may be identified by measuring the degree of the signal fluctuation excluding the fluctuations of the QRS complexes. Impulsive signals, such as non-cardiac muscles signal may be separated from ECG by using Discrete Wavelet Transform (DWT). DWT is used to decompose hierarchically discrete time signals into a series of successively lower resolution approximation signals and their associated detailed signals. At each level, the approximation and the detailed signals containing the information should be reconstructed back to the next higher resolution level, see e.g. Stephane Mallat, A Wavelet Tour of Signal Processing, Second Edition, 1999, and Robi Polikar, The Wavelet Tutorial, Rowan University, Glassboro, New Jersey, SUA,1996*.* Several wavelet bases, e.g. Daubechies (db4,db8), Symlets (sym4,sym7,sym8,sym10), Coiflets (coif5), discrete Meyer (dmey), and Biorthogonal (bior4.4), may be used for ECG enhancement, see e.g. S. Li and J. Lin, "The optimal de-noising algorithm for ECG using stationary wavelet transform, "in Proc. 2009 WRI World Cong. Computer Science Inform. Eng., vol. 6. Los Angeles, CA, pp. 469-473. The enhancement may be significant if the dilated version of the wavelet (or the scaling function) at some scale matches the shape of the signal or noise components. However, ECG non-cardiac muscles signals does not have a characteristic shape and all the above wavelet bases show a similarity with the ECG signal components at some scale. In the study a discrete Meyer (dmey) was used. A decomposition algorithm known as Mallat-tree decomposition was in the study applied to the ECG signal. The ECG signal was passed through a series of high pass filters and series of low pass filters. At each level, the high-pass filter produces the detail information d[n], while the low-pass filter associated with scaling function produces coarse approximations, a[n]. In ECG recordings with 500 Hz sampling rate, non-cardiac muscle signals are predominantly represented in the initial four details but particularly in a detail number one (D1) because of the high value of Root Mean Square (RMS) value of D1 in segments with significant non-cardiac muscle signals and because D1 is less influenced by the QRS complex, see **Figure 3****.** A 3 second epoch was used and both of HR and non-cardiac muscle activity level was expressed as the average amplitude over the last 3 minute of each exercise. Linear regression was used to establish the relationship between non-cardiac muscle signal level and HR.

### III. Result of the study and discussion

### A. Result

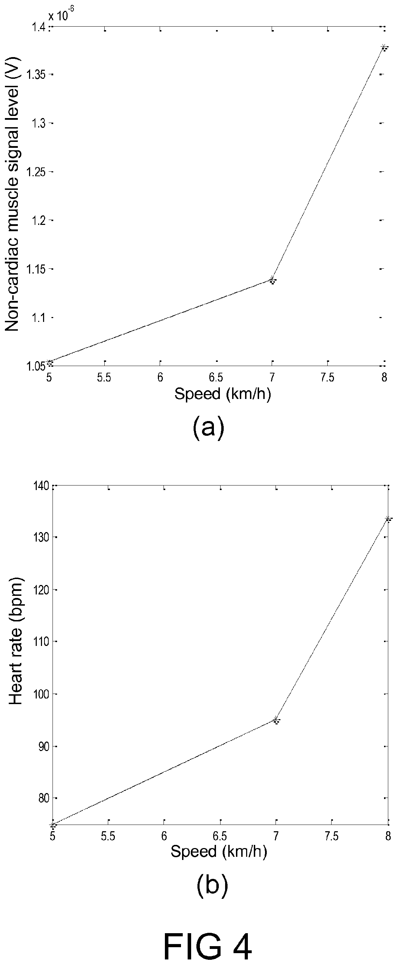

**Figure 4** illustrates the relation of the subject speed with non-cardiac muscle signal level and with heart rate. The figure shows that the heart rate and ECG-non cardiac muscle noise appears to have the same behavior while increasing speed and when changing from walking to running. Subsequent analyses were performed with the total sample.

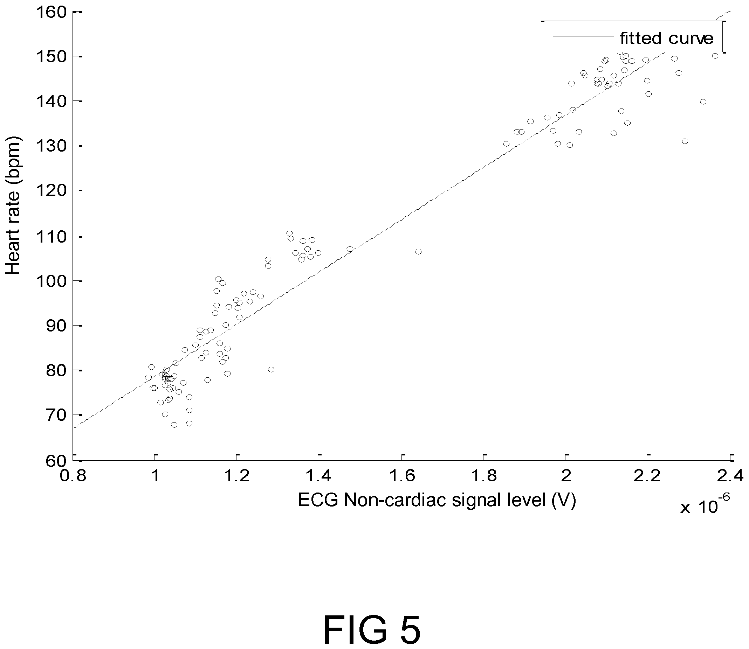

**Figure 5** illustrates an example of the relationship between non-cardiac muscle signal level and heart rate. A least square linear regression model was applied value (r=0.96, p<0.001, n=120). The variability of the regression line will increase for HR above 130 beat per minute (BPM) and only a few data points are available between the intensities that represent the walk-run transition interval 1.64-1.85 (µV) non-cardiac muscle signal.

### B. Discussion

The non-cardiac muscle signal introduced on the ECG signal may not represent the electrical activity of a special muscle but a global view of the electrical activity of both of the RA and OE, which may be supported by the fact that the electrodes are large (16 cm²) and the direct neighbourhood between RA and OE typically causes cross-talk during measurements from those locations. Moreover, because the electrodes were located on both sides of the abdomen (left and right, as shown in Figure 1), the level of non-cardiac muscle signal may not be affected by which side is activated during Stride cycle. In Figure 4 only few data points are available between the intensities that represent the walk-run transition interval (1.64-1.85) non-cardiac muscle signal. This may be explained by the fact that one of the most noticeable differences between walking and running is the existence of a flight phase in running, rather than the double support phase that occurs in walking, which suggests that muscles generate greater body support in running causing higher non-cardiac muscle signal amplitude that will affect the ECG signal and also higher muscles demand of oxygen and therefore higher heart rate. Functional investigations, systematically focusing on the activation characteristics of trunk muscles during gait are rare.

In Christoph Anders , Heiko Wagner, Christian Puta, Roland Grassme, Alexander Petrovitch, Hans-Christoph Scholle. Trunk muscle activation patterns during walking at different speeds. Journal of lectromyography and Kinesiology 17 (2007), pages 245-252*,* it is disclosed that the small and constant low amplitudes for RA at the lowest velocities increased with speed. Peaks occurred at ipsilateral heel strike and at ipsilateral as well as contralateral propulsion, but amplitudes remained at comparably low levels. For OE highest amplitude peaks were identified during contralateral propulsion phase. Smaller but distinct amplitude with contralateral heel and pad contact was observed. Peak amplitudes increased with increasing speeds. It was found that the observed amplitude peaks for OE during contralateral heel strike indicate eccentric activity. Concentric activation characteristics were evident as well: during ipsilateral heel strike, pad contact and ipsilateral propulsion phase. Moreover, it was concluded that the cumulative amplitude of all investigated trunk muscles reflects general speed dependent activation characteristics: activation peaks at ipsilateral heel strike and pad contact as well as during contralateral heel strike and propulsion phase increased with increasing speed. However, there appears to be no study that have investigated the strength of this relation.

In the present study, the evolution of the trunk muscles activity amplitude during the different phase of the Stride cycle is not of main interest but of more interest is the relation of non-cardiac muscle signal level generated by the trunk muscles, and measured by surface ECG-chest-strap electrodes, with heart activity, in particular heart rate. At low speeds trunk movements are only slight; those slight movements generate low amplitude of non-cardiac muscle signal and a small increasing in heart rate but become larger for both of them when increasing speed, see **Figure 5****.**

The differently organized trunk muscle activation patterns during the varying walking speeds result in a consistent increase of overall activity. This may argue for necessity of compensation of the higher rotational momentum forces but also for increased stabilization demands since trunk activity during gait is not essential for the walking process itself. The increase of the trunk overall activity during higher speed walking or running causes an increasing of non-cardiac muscle signal level and heart rate.

### IV. Conclusion of the study

ECG-non-cardiac muscle signal generated by trunk muscles (RA, OE) is related to the intensity of physical activity. The correlation coefficient between the heart rate and non-cardiac muscle signal levels value (r=0.96, n=120) suggest this parameter may advantageously be used to estimate physical activity. The strength of a method based on this is its ability to distinguish between HR increasing due to PA or to mental activity, e.g. stress. Also the method may not require another device or an additional hardware component but e.g. only an implementation by software in an existing device, e.g. an ECG chest-strap device.

Further studies have been performed that confirm the relationship discussed above, i.e. between HR and a ECG-non-cardiac signal that may be generated by trunk muscles, which may be referred to as ECG-Trunk Muscles signals amplitude (ECG-TMSA). The further studies have confirmed that ECG signals and the relationship after it has been established may be used to estimate, the PA level. Also, as ECG-TMSA is independent on mental activities and may thus be used with an established relationship to estimate PA without influence of e.g. mental stress. It has further been found that ECG-TMSA and the relationship may even be used to distinguish between e.g. sitting and walking actions, and even distinguishing between moving on site and walking.

Since an ECG signal is used it is very convenient to identify HR from the same ECG signal and thereby be able to e.g. remove influence of mental stress in HR or to find the contribution of mental stress in the HR. For example, the level of PA may be estimated from the established relationship and the ECG signal, and HR be determined from the same ECG signal. The estimated level of PA may correspond to an estimated heart rate independent of mental stress. By e.g. comparing the HR determined from the ECG signal with the estimated heart rate, heart rate resulting from mental stress may be identified and/or determined.

Hence it is enabled, based on the ECG signal, to conveniently determine and/or distinguish HR resulting from PA and/or from mental stress.

The relationship may be used as and/or seen as representing a new physiological variable. Based on the relationship methods and algorithms may be implemented for use in real life in situations where presently known methods cannot be used or is less convenient to use e.g. for distinguishing PA and/or HR resulting from an individual walking slowly, moving on site, and/or being mentally stressed.

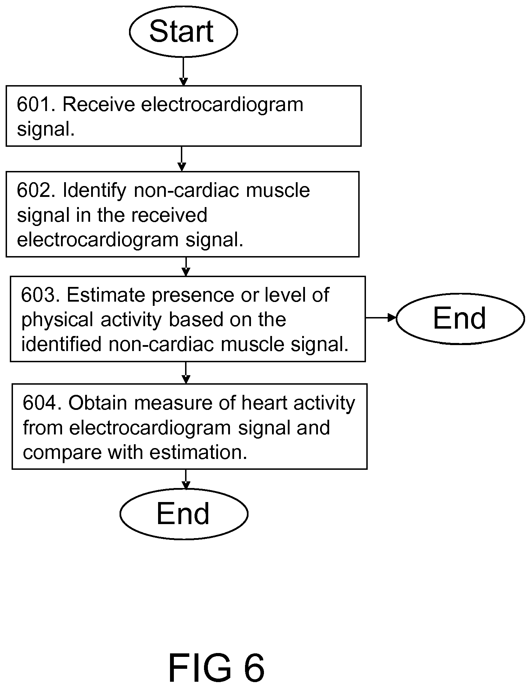

At least partly based on the above study, embodiments herein relating to a method for estimating physical activity of an individual based on electrocardiogram, will now be further elaborated and described with reference to the flowchart depicted in **Figure 6****.** The method comprises the following actions, which actions may be taken in any suitable order. Further, actions may be combined.

### Action 601

An electrocardiogram signal originating from the individual is received.

The non-cardiac muscle signal may preferably comprise signals generated by trunk muscles.

In some embodiments the electrocardiogram signal is received from electrodes, i.e. at least two, positioned on the chest of the individual under the pectoral muscles and preferably distributed on both of the left and the right body halves of the individual. The electrodes may be comprised in a chest-strap that may be bipolar and attached to the chest of the individual, such as by the chest-strap 101 shown in Figure 1. However, as realized by the skilled person, the electrodes may also be attached by other means, e.g. by glue or by other conventional means for attaching electrodes for electrocardiogram registration.

This action may fully or partly correspond to what was disclosed above under "II Method of the study" / "C. Instrumentation" / "ECG monitor and Electrode positions".

### Action 602

A non-cardiac muscle signal in the received electrocardiogram signal is identified.

In some embodiments the identification of the non-cardiac muscle signal comprises to measure a degree of fluctuations in the electrocardiogram signal excluding fluctuations of QRS complexes.

This action may fully or partly correspond to what was disclosed above under "II Method of the study" / "C. Instrumentation" / "Data processing".

### Action 603

A presence or a level of physical activity of the individual based on the identified non-cardiac muscle signal is estimated. In some embodiments the estimated presence or level of physical activity is further based on an established relationship between non-cardiac muscle signal levels, typically amplitudes, and measures of physical activity. The established relationship may be linear and may be represented by a line, which in turn may be represented by a value defined by and/or corresponding to the slope of the line. The measures of physical activity may comprise measures of heart activity. The measures of heart activity may comprise measures of heart rate.

In some embodiments there may be a relationship that is not only, or has not only been, established for the individual alone. The relationship may instead have been established for a profile that is the same for a group of individuals and for which profile the same or similar relationship may apply. The individual as such may or may not be part of the group. For example may the profile be based on age, sex, body fat percentage and/or body mass index of individuals, just to mention some examples. An individual may then be identified as belonging to a certain profile and then a relationship that has been established for that profile is used. A group of individuals corresponding to the profile may have been used to establish the relationship, e.g. as an average of individual relationships, e.g. represented by values, established for each one of the individuals.

In some embodiments, a duration and/or frequency of physical activity associated with the estimated presence or level of physical activity are determined. For example, the duration and/or frequency may be measured, and thereby be determined, as long as there is estimated presence of physical activity. The duration and/or frequency of physical activity may be determined based on the identified non-cardiac muscle signal.

This action may fully or partly correspond to what was disclosed above under "II Result of the study" and discussion and under "IV Conclusion of the study". Establishment of the relationship for an individual will also be further discussed below.

### Action 604

This is an optional action for embodiments herein. The estimated presence or level of physical activity corresponds to an estimated measure of heart activity. A measure of heart activity is obtained from the electrocardiogram signal and compared with the estimated measure of heart activity. Thereby reduction of influence of physical activity in the measure of heart activity and/or due to mental stress of the individual is enabled.

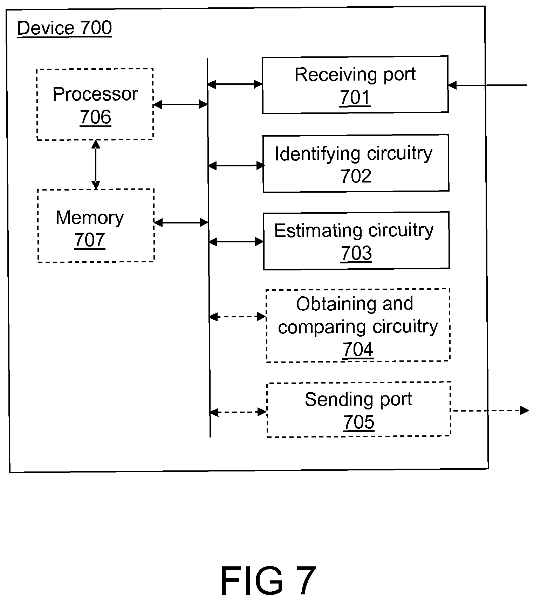

To perform the actions above for estimating the physical activity of the individual based on electrocardiogram, there may be provided a device 700, according to embodiments herein, which device may comprise an arrangement schematically depicted in **Figure 7****.**

The device 700 comprises **a receiving port 701,** configured to receive the electrocardiogram signal originating from the individual. In some embodiments the receiving port 701 is further configured to receive the electrocardiogram signal from electrodes, i.e. at least two, positioned on the chest of the individual under the pectoral muscles and preferably distributed on both of the left and the right body halves of the individual.

The device 700 further comprises **an identifying circuitry 702,** configured to identify the non-cardiac muscle signal in the received electrocardiogram signal. In some embodiments, the identifying circuitry 702 is further configured to measure the degree of fluctuations in the electrocardiogram signal excluding fluctuations of QRS complexes.

Moreover, the device 700 comprises **an estimating circuitry 703,** configured to estimat the presence or the level of physical activity of the individual based on the identified non-cardiac muscle signal. In some embodiments the estimating circuitry 703 is further configured to estimate the presence or the level of physical activity based on an established relationship between non-cardiac muscle signal levels and measures of physical activity. In some embodiments, the estimating circuitry 703 is further configured to determine the duration and/or frequency of physical activity associated with the estimated presence or level of physical activity.

In some embodiments, the estimated presence or level of physical activity corresponds to an estimated measure of heart activity, and the device 700 further comprises **an obtaining and comparing circuitry 704,** configured to obtain the measure of heart activity from the electrocardiogram signal and to compare the obtained measure with the estimated measure of heart activity, thereby enabling reduction of influence of physical activity in the measure of heart activity and/or due to mental stress of the individual.

The device 700 may further comprise **a sending port 705** that may be configured to send information to and be involved in communication with another device, e.g. a separate device for using and/or presenting results of the estimated presence or level of physical activity device.

The embodiments of the device 700 may be implemented through one or more processors, such as **a processor 706** in the device 700 depicted in Figure 7, together with computer program code for performing the functions and actions of embodiments herein. In some embodiments the circuitry discussed above may be fully or partially implemented by the processor 706.

The computer program code mentioned above may also be provided as a computer program product, for instance in the form of a data carrier carrying computer program code for performing the embodiments herein when being loaded into the device 700. One such carrier may be in the form of a CD or DVD. It is however feasible with other data carriers such as a memory stick, memory card or hard drive. The computer program code may furthermore be provided as pure program code on a server for download to the device 700. The computer program code may furthermore be provided in the form of a data file or files on, or available through, a server for download. The file or files may be executable files for direct or indirect download to and execution on the device 700, or may be for intermediate download and compilation to make them executable before download to and for execution in the device 700. The server may be accessible over a computer network, such as the Internet, and may e.g. be a web or ftp server.

The device 700 may further comprise a **memory 707** comprising one or more memory units. The memory 707 may be arranged to store data, for example received signals, intermediate values and/or results, configurations and/or applications to perform the method when being executed in the device 700.

Those skilled in the art will also appreciate that the circuitries and/or ports 701-705 may refer to a combination of analog and digital circuits, and/or one or more processors configured with software and/or firmware (e.g., stored in memory) that, when executed by the one or more processors such as the processor 706, perform as described above. One or more of these processors, as well as the other digital hardware, may be included in a single application-specific integrated circuit (ASIC), or several processors and various digital hardware may be distributed among several separate components, whether individually packaged or assembled into a system-on-a-chip (SoC).

In some embodiments the device 700 may correspond to, or be comprised in, a personal computer, a chest-strap with electrodes, a Holter device, a wrist watch with heart activity monitoring capability, just to mention some examples. The device 700, especially when it is of a type to be worn by the individual, may further have logging and storage capability, e.g. storing in the memory 770. The logging and storing may be of the estimated level of physical activity of the individual during a time period, or of data based on the estimated presence or level of physical activity. Also measured heart rate from and/or the electrocardiogram signal as such may be logged and stored. What have been logged and stored may later be used to find out about the physical activity, heart rate, mental stress, and changes thereof, for the individual dung the time period, and even to distinguish whether the physical activity pertains to walking or moving on site.

Implementation of the device 700 may involve updating a software of an existing device.

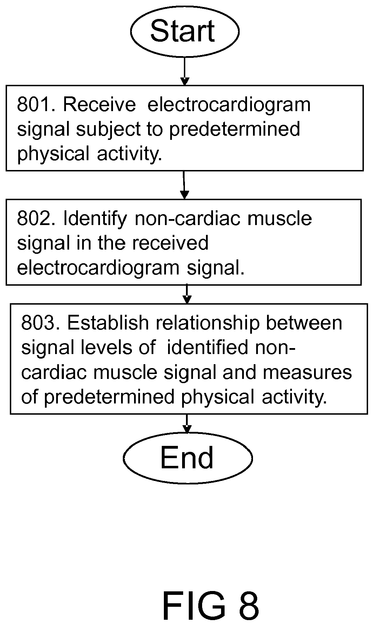

At least partly based on the above study, embodiments herein relating to a method for supporting estimation of physical activity of an individual based on electrocardiogram, will now be further elaborated and described with reference to the flowchart depicted in **Figure 8****.** The method comprises the following actions, which actions may be taken in any suitable order. Further, actions may be combined.

### Action 801

An electrocardiogram signal is received. The electrocardiogram signal originates from the individual when the individual was subjected to a predetermined physical activity.

The non-cardiac muscle signal may preferably comprise signals generated by trunk muscles.

In some embodiments the electrocardiogram signal is received from electrodes, i.e. at least two, positioned on the chest of the individual under the pectoral muscles and preferably distributed on both of the left and the right body halves of the individual. The electrodes may be comprised in a chest-strap that may be bipolar and attached to the chest of the individual, such as by the chest-strap 101. However, as realized by the skilled person, the electrodes may also be attached by other means, e.g. by glue or by other conventional means for attaching electrodes for electrocardiogram registration.

This action may fully or partly correspond to what was disclosed above under "II Method of the study" / "C. Instrumentation" / "ECG monitor and Electrode positions" and under Action 601.

### Action 802

A non-cardiac muscle signal is identified in the received electrocardiogram signal.

In some embodiments the identification of the non-cardiac muscle signal comprises to measure a degree of fluctuations in the electrocardiogram signal excluding fluctuations of QRS complexes.

This action may fully or partly correspond to what was disclosed above under "II Method of the study" / "C. Instrumentation" / "Data processing" and under Action 602.

### Action 803

A relationship between signal levels of the identified non-cardiac muscle signal and measures of the predetermined physical activity is established. The established relationship may be used as the established relationship discussed above in connection with Figures 6 and 7.

In some embodiment linear regression may be used to establish the relationship. The measures of the predetermined physical activity may comprise measures of heart activity. The measures of heart activity may preferably be extracted from the electrocardiogram signal. The measures of heart activity may comprise measures of heart rate.

This action may fully or partly correspond to what was disclosed above under "II Result of the study" and discussion and under "IV Conclusion of the study".

As should be understood, actions 801-803 may be performed separate from action 601-604, typically in beforehand at some point in time, to establish the relationship. The established relationship is may then be used for the individual in Actions 601-604 to estimate the presence or level of physical activity. Note that, as already mentioned, it is not needed to be the same individual or individuals involved in establishing the relationship as the individual for which the established relationship is used to estimate the presence or level of physical activity.

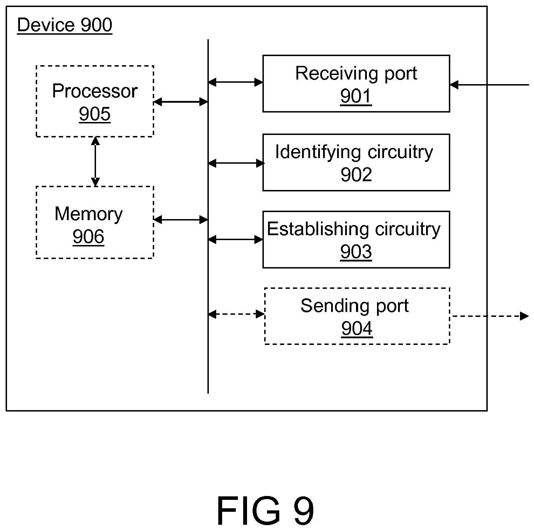

To perform the actions above for supporting estimation of the physical activity of the individual based on electrocardiogram, there may be provided a device 900, according to embodiments herein, which device may comprise an arrangement schematically depicted in **Figure 9****.**

The device 900 comprises **a receiving port 901,** configured to receive the electrocardiogram signal originating from the individual when the individual was subjected to a predetermined physical activity. In some embodiments, the receiving port 901 is further configured to receive the electrocardiogram signal from electrodes, i.e. at least two, positioned on the chest of the individual under the pectoral muscles and preferably distributed on both of the left and the right body halves of the individual.

The device 900 further comprises **an identifying circuitry 902,** configured to identify the non-cardiac muscle signal in the received electrocardiogram signal. In some embodiments, the identifying circuitry 902 is further configured to measure the degree of fluctuations in the electrocardiogram signal excluding fluctuations of QRS complexes.

Moreover, the device 900 comprises **an establishing circuitry 903,** configured to establish the relationship between the signal levels of the identified non-cardiac muscle signal and the measures of the predetermined physical activity. In some embodiments, the establishing circuitry 903 is further configured to use linear regression to establish the relationship.

The device 900 may further comprise **a sending port 904** that may be configured to send the established relationship, and/or be involved in other communication with, another device that may be a separate device, e.g. the device 700 or a computer. Note that the device 700 and device 900 may, but not necessarily, be integrated in the same physical device.

The embodiments of the device 900 may be implemented through one or more processors, such as **a processor 905** in the device 900 depicted in Figure 9, together with computer program code for performing the functions and actions of embodiments herein. In some embodiments the circuitry discussed above may be fully or partially implemented by the processor 905.

The computer program code mentioned above may also be provided as a computer program product, for instance in the form of a data carrier carrying computer program code for performing the embodiments herein when being loaded into the device 900. One such carrier may be in the form of a CD or DVD. It is however feasible with other data carriers such as a memory stick, memory card or hard drive. The computer program code may furthermore be provided as pure program code on a server for download to the device 900. The computer program code may furthermore be provided in the form of a data file or files on, or available through, a server for download. The file or files may be executable files for direct or indirect download to and execution on the device 900, or may be for intermediate download and compilation to make them executable before download to and for execution in the device 900. The server may be accessible over a computer network, such as the Internet, and may e.g. be a web or ftp server.

The device 900 may further comprise a **memory 906** comprising one or more memory units. The memory 907 may be arranged to store data, for example received signals, intermediate values and/or results, configurations and/or applications to perform the method when being executed in the device 900.

Those skilled in the art will also appreciate that the circuitries and ports 901-904 may refer to a combination of analog and digital circuits, and/or one or more processors configured with software and/or firmware (e.g., stored in memory) that, when executed by the one or more processors such as the processor 905, perform as described above. One or more of these processors, as well as the other digital hardware, may be included in a single application-specific integrated circuit (ASIC), or several processors and various digital hardware may be distributed among several separate components, whether individually packaged or assembled into a system-on-a-chip (SoC).

In some embodiments the device 900 may correspond to, or be comprised in, a personal computer, a chest-strap strap with electrodes, a Holter device, a wrist watch with heart rate monitoring capability, just to mention some examples.

Implementation of the device 900 may involve updating a software of an existing device.

The method for supporting estimation of physical activity, i.e. the method ending with the established relationship as discussed above in connection with Figure 8, may be performed in a calibration phase. The method for estimating physical activity, i.e. as discussed above in connection with Figure 6, may then be performed in a subsequent measurement phase where the established relationship from the calibration phase may be used. In the measurement phase, the received electrocardiogram signal originating from the individual is typically another electrocardiogram signal than during the calibration phase. In the measurement phase, the identified non-cardiac muscle signal may be a corresponding but not the same non-cardiac muscle signal as in the calibration phase, as may be realized since the electrocardiogram signals are typically not the same. Preferably, but not necessarily as mentioned above, the individual is the same individual, and preferably the electrodes are positioned substantially at the same positions, during the measurement phase as during the calibration phase. When the established relationship is used to estimate presence or level of physical activity, the identified non-cardiac muscle signal and the established relationship may result in the estimated presence or level of physical activity in the form of an estimated measure of physical activity.

The relationship may advantageously be represented by a line and thus be considered substantially linear. As should be understood by the skilled person, also other higher-order relationships that respectively is fitted to the data points and e.g. corresponds to a curve that is close to the line, e.g. close to the line shown in Figure 5, may be used to represent the relationship, although such higher-order relationships are mathematically not linear.

## Claims

1. A method, performed by a device (700), for estimating physical activity of an individual based on electrocardiogram, wherein the method comprises:
- *receiving (601)* an electrocardiogram signal originating from the individual;
- *identifying (602)* a non-cardiac muscle signal in the received electrocardiogram signal, wherein the identifying of the non-cardiac muscle signal comprises measuring a degree of fluctuations in the electrocardiogram signal excluding fluctuations of QRS complexes;
- *estimating (603)* a presence or a level of physical activity of the individual, wherein the estimated presence or level of physical activity corresponds to an estimated measure of heart activity and which estimation is based on the identified non-cardiac muscle signal and on an established relationship between non-cardiac muscle signal levels and measures of physical activity, which measures comprise measures of heart activity; and
- *obtaining (604)* a measure of heart activity from the electrocardiogram signal and comparing with the estimated measure of heart activity, thereby enabling reduction of influence of physical activity in the measure of heart activity and/or due to mental stress of the individual.

2. The method as claimed in claim 1, wherein the non-cardiac muscle signal comprises signals generated by trunk muscles.

3. The method as claimed in any one of claims 1-2, wherein the electrocardiogram signal is received from electrodes positioned on the chest of the individual under the pectoral muscles and preferably distributed on both of the left and the right body halves of the individual.

4. The method as claimed in claim 3, wherein the electrodes are comprised in a chest-strap (101) attached to the chest of the individual.

5. A method, performed by a device (900), for supporting estimation of physical activity of an individual based on electrocardiogram, wherein the method comprises:
- *receiving (801)* an electrocardiogram signal originating from the individual when the individual was subjected to a predetermined physical activity;
- *identifying (802)* a non-cardiac muscle signal in the received electrocardiogram signal, wherein the identifying of the non-cardiac muscle signal comprises measuring a degree of fluctuations in the electrocardiogram signal excluding fluctuations of QRS complexes; and
- *establishing (803),* by using linear regression, a relationship between signal levels of the identified non-cardiac muscle signal and measures of the predetermined physical activity, which measures comprise measures of heart activity extracted from the electrocardiogram signal.

6. The method as claimed in claim 5, wherein the non-cardiac muscle signal comprises signals generated by trunk muscles.

7. The method as claimed in any one of claims 5-6, wherein the electrocardiogram signal is received from electrodes positioned on the chest of the individual under the pectoral muscles and preferably distributed on both of the left and the right body halves of the individual.

8. The method as claimed in claim 7, wherein the electrodes are comprised in a chest-strap (101) attached to the chest of the individual.

9. A computer program comprising code that when being loaded into a device (700; 900) performs the method according to any one of claims 1-4 or any one of claims 5-8.

10. A data carrier comprising the computer program according to claim 9.

11. A device (700) for estimating physical activity of an individual based on electrocardiogram, wherein the device (700) comprises:
a *receiving port (701),* configured to receive an electrocardiogram signal originating from the individual;
*an identifying circuitry (702),* configured to identify a non-cardiac muscle signal in the received electrocardiogram signal; and
*an estimating circuitry (703),* configured to estimate a presence or a level of physical activity of the individual,
wherein the device (700) is **characterized by**
that said estimated presence or level of physical activity corresponds to an estimated measure of heart activity and which estimation is based on the identified non-cardiac muscle signal and on an established relationship between non-cardiac muscle signal levels and measures of physical activity, which measures comprise measures of heart activity;
wherein the identifying circuitry (702) is further configured to measure a degree of fluctuations in the electrocardiogram signal excluding fluctuations of QRS complexes, and by that the device (900) further comprises:
*an obtaining and comparing circuitry (704),* configured to obtain a measure of heart activity from the electrocardiogram signal and to compare the obtained measure with the estimated measure of heart activity, thereby enabling reduction of influence of physical activity in the measure of heart activity and/or due to mental stress of the individual.

12. A device (900) for supporting estimation of physical activity of an individual based on electrocardiogram, wherein the device (900) comprises:
a *receiving port (901),* configured to receive an electrocardiogram signal originating from the individual when the individual was subjected to a predetermined physical activity; and
*an identifying circuitry (902),* configured to identify a non-cardiac muscle signal in the received electrocardiogram signal;
wherein the device (900) is **characterized by**
that the identifying circuitry (902) is configured to measure a degree of fluctuations in the electrocardiogram signal excluding fluctuations of QRS complexes, and by that the device (900) further comprises:
*an establishing circuitry (903)* configured to use linear regression to establish a relationship between signal levels of the identified non-cardiac muscle signal and measures of the predetermined physical activity, which measures comprise measures of heart activity extracted from the electrocardiogram signal.

13. A personal computer, a chest strap with electrodes, a Holter device or a wrist watch with heart activity monitoring capability, comprising the device (700; 900) according to any one of claims 11-12.

## Patentansprüche

1. Verfahren, das von einer Vorrichtung (700) durchgeführt wird, zum Schätzen der körperlichen Aktivität eines Individuums basierend auf einem Elektrokardiogramm, wobei das Verfahren Folgendes umfasst:
- *Empfangen (601)* eines Elektrokardiogrammsignals, das von dem Individuum stammt;
- *Kennzeichnen (602)* eines nicht-kardialen Muskelsignals in dem empfangenen Elektrokardiogrammsignal, wobei das Kennzeichnen des nicht-kardialen Muskelsignals das Messen eines Grads von Schwankungen in dem Elektrokardiogrammsignal unter Ausschluss von Schwankungen von QRS-Komplexen umfasst;
- *Schätzen (603)* eines Vorhandenseins oder eines Niveaus der körperlichen Aktivität des Individuums, wobei das geschätzte Vorhandensein oder Niveau der körperlichen Aktivität einer geschätzten Messgröße der Herzaktivität entspricht, und wobei die Schätzung auf dem gekennzeichneten nicht-kardialen Muskelsignal und auf einem festgelegten Verhältnis zwischen nicht-kardialen Muskelsignalniveaus und Messgrößen der körperlichen Aktivität basiert, wobei die Messgrößen Messgrößen der Herzaktivität umfassen; und
- *Erlangen (604)* einer Messgröße der Herzaktivität anhand des Elektrokardiogrammsignals und Vergleichen mit der geschätzten Messgröße der Herzaktivität, wodurch eine Verringerung des Einflusses der körperlichen Aktivität auf die Messgröße der Herzaktivität und/oder aufgrund der mentalen Belastung des Individuums ermöglicht wird.

2. Verfahren nach Anspruch 1, wobei das nicht-kardiale Muskelsignal Signale umfasst, die von Rumpfmuskeln erzeugt werden.

3. Verfahren nach einem der Ansprüche 1-2, wobei das Elektrokardiogrammsignal von Elektroden empfangen wird, die auf der Brust des Individuums unter den Brustmuskeln positioniert sind und vorzugsweise sowohl auf der linken als auch der rechten Körperhälfte des Individuums verteilt sind.

4. Verfahren nach Anspruch 3, wobei die Elektroden in einem Brustgurt (101) enthalten sind, der an der Brust des Individuums befestigt ist.

5. Verfahren, das von einer Vorrichtung (900) durchgeführt wird, zum Unterstützen der Schätzung der körperlichen Aktivität eines Individuums basierend auf einem Elektrokardiogramm, wobei das Verfahren Folgendes umfasst:
- *Empfangen (801)* eines Elektrokardiogrammsignals, das von dem Individuum stammt, wenn das Individuum einer vorbestimmten körperlichen Aktivität ausgesetzt wurde;
- *Kennzeichnen (802)* eines nicht-kardialen Muskelsignals in dem empfangenen Elektrokardiogrammsignal, wobei das Kennzeichnen des nicht-kardialen Muskelsignals das Messen eines Grads von Schwankungen in dem Elektrokardiogrammsignal unter Ausschluss von Schwankungen von QRS-Komplexen umfasst; und
- *Festlegen (803)* durch Verwenden einer linearen Regression eines Verhältnisses zwischen Signalpegeln des gekennzeichneten nicht-kardialen Muskelsignals und Messgrößen der vorbestimmten körperlichen Aktivität, wobei die Messgrößen Messgrößen der Herzaktivität, die aus dem Elektrokardiogrammsignal extrahiert werden, umfassen.

6. Verfahren nach Anspruch 5, wobei das nicht-kardiale Muskelsignal Signale umfasst, die von Rumpfmuskeln erzeugt werden.

7. Verfahren nach einem der Ansprüche 5 - 6, wobei das Elektrokardiogrammsignal von Elektroden empfangen wird, die auf der Brust des Individuums unter den Brustmuskeln positioniert sind und vorzugsweise sowohl auf der linken als auch der rechten Körperhälfte des Individuums verteilt sind.

8. Verfahren nach Anspruch 7, wobei die Elektroden in einem Brustgurt (101) enthalten sind, der an der Brust des Individuums befestigt ist.

9. Computerprogramm, das einen Code umfasst, der, wenn er in eine Vorrichtung (700; 900) geladen wird, das Verfahren nach einem der Ansprüche 1 - 4 oder einem der Ansprüche 5 - 8 durchführt.

10. Datenträger, der das Computerprogramm nach Anspruch 9 umfasst.

11. Vorrichtung (700) zum Schätzen einer körperlichen Aktivität eines Individuums basierend auf einem Elektrokardiogramm, wobei die Vorrichtung (700) Folgendes umfasst:
*einen Empfangsanschluss (701),* der konfiguriert ist, um ein Elektrokardiogrammsignal zu empfangen, das von dem Individuum stammt;
*eine Kennzeichnungsschaltung (702),* die konfiguriert ist, um ein nicht-kardiales Muskelsignal in dem empfangenen Elektrokardiogrammsignal zu kennzeichnen; und
*eine Schätzungsschaltung (703),* die konfiguriert ist, um ein Vorhandensein oder
ein Niveau einer körperlichen Aktivität des Individuums zu schätzen,
wobei die Vorrichtung (700) **dadurch gekennzeichnet ist, dass** das geschätzte Vorhandensein oder Niveau einer körperlichen Aktivität einer geschätzten Messgröße der Herzaktivität entspricht, und wobei die Schätzung auf dem gekennzeichneten nicht-kardialen Muskelsignal und auf einem festgelegten Verhältnis zwischen nicht-kardialen Muskelsignalpegeln und Messgrößen der körperlichen Aktivität basiert, wobei die Messgrößen Messgrößen der Herzaktivität umfassen;
wobei die Kennzeichnungsschaltung (702) ferner konfiguriert ist, um einen Grad von Schwankungen in dem Elektrokardiogrammsignal unter Ausschluss von Schwankungen von QRS-Komplexen zu messen, und dass die Vorrichtung (900) ferner Folgendes umfasst:
*eine Erlangungs- und Vergleichsschaltung (704),* die konfiguriert ist, um eine Messgröße der Herzaktivität anhand des Elektrokardiogrammsignals zu erlangen und die erlangte Messgröße mit der geschätzten Messgröße der Herzaktivität zu vergleichen, wodurch eine Verringerung des Einflusses der körperlichen Aktivität auf die Messgröße der Herzaktivität und/oder aufgrund der mentalen Belastung des Individuums ermöglicht wird.

12. Vorrichtung (900) zum Unterstützen der Schätzung der körperlichen Aktivität eines Individuums basierend auf einem Elektrokardiogramm, wobei die Vorrichtung (900) Folgendes umfasst:
*einen Empfangsanschluss (901),* der konfiguriert ist, um ein Elektrokardiogrammsignal zu empfangen, das von dem Individuum stammt, wenn das Individuum einer vorbestimmten körperlichen Aktivität ausgesetzt wurde; und
eine Kennzeichnungsschaltung (902), die konfiguriert ist, um ein nicht-kardiales Muskelsignal in dem empfangenen Elektrokardiogrammsignal zu kennzeichnen;
wobei die Vorrichtung (900) **dadurch gekennzeichnet ist, dass** die *Kennzeichnungsschaltung (902)* konfiguriert ist, um einen Grad von Schwankungen in dem Elektrokardiogrammsignal unter Ausschluss von Schwankungen von QRS-Komplexen zu messen, und dass die Vorrichtung (900) ferner Folgendes umfasst:
*eine Festlegungsschaltung (903),* die konfiguriert ist, um eine lineare Regression zum Festlegen eines Verhältnisses zwischen Signalpegeln des gekennzeichneten nicht-kardialen Muskelsignals und Messgrößen der vorbestimmten körperlichen Aktivität festzulegen, wobei die Messgrößen Messgrößen der Herzaktivität, die aus dem Elektrokardiogrammsignal extrahiert werden, umfassen.

13. Personal Computer, Brustgurt mit Elektroden, Holter-Vorrichtung oder Armbanduhr mit Herzaktivitätsüberwachungsfähigkeit umfassend die Vorrichtung (700; 900) nach einem der Ansprüche 11-12.

## Revendications

1. Procédé, mis en oeuvre par un dispositif (700), pour estimer une activité physique d'un individu sur la base d'un électrocardiogramme, dans lequel le procédé comprend les étapes consistant à :
- *recevoir* (601) un signal d'électrocardiogramme provenant de l'individu;
- *identifier* (602) un signal de muscle non cardiaque dans le signal d'électrocardiogramme reçu, dans lequel l'identification du signal de muscle non cardiaque comprend la mesure d'un degré de fluctuations dans le signal d'électrocardiogramme à l'exclusion de fluctuations de complexes QRS;
- *estimer* (603) une présence ou un niveau d'activité physique de l'individu, dans lequel la présence ou le niveau estimé(e) d'activité physique correspond à une mesure estimée d'activité cardiaque, et laquelle estimation est basée sur le signal de muscle non cardiaque identifié et sur une relation établie entre des niveaux de signal de muscle non cardiaque et des mesures d'activité physique, lesquelles mesures comprennent des mesures d'activité cardiaque ; et
- *obtenir* (604) une mesure d'activité cardiaque à partir du signal d'électrocardiogramme et comparer avec la mesure d'activité cardiaque estimée, en permettant ainsi une réduction de l'influence d'une activité physique dans la mesure de l'activité cardiaque et/ou en raison d'un stress mental de l'individu.

2. Procédé selon la revendication 1, dans lequel le signal de muscle non cardiaque comprend des signaux générés par des muscles du tronc.

3. Procédé selon l'une quelconque des revendications 1 et 2, dans lequel le signal d'électrocardiogramme est reçu à partir d'électrodes positionnées sur la poitrine de l'individu sous les muscles pectoraux et de préférence réparties à la fois sur les moitiés de corps gauche et droite de l'individu.

4. Procédé selon la revendication 3, dans lequel les électrodes sont incluses dans une sangle de poitrine (101) attachée à la poitrine de l'individu.

5. Procédé, mis en oeuvre par un dispositif (900), pour supporter une estimation d'activité physique d'un individu sur la base d'un électrocardiogramme, dans lequel le procédé comprend les étapes consistant à :
- *recevoir* (801) un signal d'électrocardiogramme provenant de l'individu lorsque l'individu a été soumis à une activité physique prédéterminée ;
- *identifier* (802) un signal de muscle non cardiaque dans le signal d'électrocardiogramme reçu, dans lequel l'identification du signal de muscle non cardiaque comprend la mesure d'un degré de fluctuations du signal d'électrocardiogramme à l'exclusion de fluctuations de complexes QRS ; et
- *établir* (803), en utilisant une régression linéaire, une relation entre des niveaux de signal du signal de muscle cardiaque identifié et des mesures de l'activité physique prédéterminée, lesquelles mesures comprennent des mesures d'activité cardiaque extraites du signal d'électrocardiogramme.

6. Procédé selon la revendication 5, **caractérisé en ce que** le signal du muscle non cardiaque comprend des signaux générés par des muscles du tronc.

7. Procédé selon l'une quelconque des revendications 5 et 6, dans lequel le signal d'électrocardiogramme est reçu à partir d'électrodes positionnées sur la poitrine de l'individu sous les muscles pectoraux et de préférence réparties à la fois sur les moitiés de corps gauche et droite de l'individu.

8. Procédé selon la revendication 7, **caractérisé en ce que** les électrodes sont incluses dans une sangle de poitrine (101) attachée à la poitrine de l'individu.

9. Programme informatique comprenant un code qui, lorsqu'il est chargé dans un dispositif (700; 900), met en oeuvre le procédé selon l'une quelconque des revendications 1 à 4 ou l'une quelconque des revendications 5 à 8.

10. Support de données comprenant le programme informatique selon la revendication 9.

11. Dispositif (700) pour estimer une activité physique d'un individu sur la base d'un électrocardiogramme, dans lequel le dispositif (700) comprend :
*un orifice de réception* (701), configuré pour recevoir un signal d'électrocardiogramme provenant de l'individu ;
*un circuit d'identification* (702), configuré pour identifier un signal de muscle non cardiaque dans le signal d'électrocardiogramme reçu ; et
*un circuit d'estimation* (703), configuré pour estimer une présence ou un niveau d'activité physique de l'individu,
dans lequel le dispositif (700) est **caractérisé en ce que**
ladite présence ou ledit niveau d'activité physique estimé(e) correspond à une mesure estimée d'activité cardiaque, et laquelle estimation est basée sur le signal de muscle non-cardiaque identifié et sur une relation établie entre des niveaux de signal de muscle non cardiaque et des mesures d'activité physique, lesquelles mesures comprennent des mesures d'activité cardiaque ;
dans lequel le circuit d'identification (702) est en outre configuré pour mesurer un degré de fluctuations dans le signal d'électrocardiogramme, à l'exclusion de fluctuations de complexes QRS, et **en ce que** le dispositif (900) comprend en outre :
*un circuit d'obtention et de comparaison* (704), configuré pour obtenir une mesure d'activité cardiaque à partir du signal d'électrocardiogramme et pour comparer la mesure obtenue avec la mesure estimée d'activité cardiaque, en permettant ainsi une réduction de l'influence d'une activité physique dans la mesure de l'activité cardiaque et/ou en raison d'un stress mental de l'individu.

12. Dispositif (900) destiné à supporter une estimation d'activité physique d'un individu sur la base d'un électrocardiogramme, dans lequel le dispositif (900) comprend :
*un orifice de réception* (901), configuré pour recevoir un signal d'électrocardiogramme provenant de l'individu lorsque l'individu a été soumis à une activité physique prédéterminée ; et
*un circuit d'identification* (902), configuré pour identifier un signal de muscle non cardiaque dans le signal d'électrocardiogramme reçu ;
dans lequel le dispositif (900) est **caractérisé en ce que**
le circuit d'identification (902) est configuré pour mesurer un degré de fluctuations dans le signal d'électrocardiogramme, à l'exclusion de fluctuations de complexes QRS, et **en ce que** le dispositif (900) comprend en outre :
*un circuit d'établissement* (903) configuré pour utiliser une régression linéaire pour établir une relation entre des niveaux de signal du signal de muscle non cardiaque identifié et des mesures de l'activité physique prédéterminée, lesquelles mesures comprennent des mesures d'activité cardiaque extraites du signal d'électrocardiogramme.

13. Ordinateur personnel, sangle thoracique munie d'électrodes, dispositif Holter ou montre-bracelet ayant une capacité de surveillance d'activité cardiaque, comprenant le dispositif (700 ; 900) selon l'une quelconque des revendications 11 et 12.
